# EUROPEAN PATENT APPLICATION

(11) **EP 4 721 652 A1**
(43) Date of publication of application: **08.04.2026**
(21) Application number: 24881339.6
(22) Date of filing: 24.09.2024
(51) Int. Cl.: A61B 5/0205, A61B 5/256, A61B 5/28, A61B 5/00

(54) **INTELLIGENT FINGER RING**

(30) Priority: 27.10.2023 CN 202322922267 U
(71) Applicant: Anhui Huami Health Technology Co., Ltd., Hefei, Anhui 230088 (CN)
(72) Inventor: ZOU, Aijun, Hefei, Anhui 230088 (CN); SHAO, Mingbao, Hefei, Anhui 230088 (CN); LI, Tongchun, Hefei, Anhui 230088 (CN)
(74) Representative: Dai, Simin
(86) International application number: PCT/CN2024/120586
(87) International publication number: WO 2025/086973

(57) **Abstract**

A smart ring device (100), including: a housing (10) including an outer ring (11), an inner ring (12), and an accommodation space (13) located between the outer ring (11) and the inner ring (12); at least one electronic component (20), disposed in the accommodation space (13); and a first electrode (30) and a second electrode (40), spaced apart along a circumferential direction of the housing (10) and electrically connected to the at least one electronic component (20).

## Description

### TECHNICAL FIELD

The present disclosure relates to the technical field of smart wearable devices, and more particularly, to smart ring devices.

### BACKGROUND

With the development of smart wearable technology, smart ring devices have evolved to integrate more and more functions. Due to their ergonomic design for finger-wearing, smart ring devices have emerged as a promising trend for personal health monitoring.

Therefore, how to achieve personal health monitoring using smart ring devices has become a research hotspot in the field.

### SUMMARY

The embodiments of the present disclosure provides a smart ring device, including: an annular housing including an outer ring, an inner ring, and an accommodation space between the outer ring and the inner ring; at least one electronic component disposed in the accommodation space; and a first electrode and a second electrode, spaced apart along a circumferential direction of the inner surface of the annular housing and electrically connected to the at least one electronic component.

The first electrode and the second electrode may be disposed on the inner surface of the inner ring and arranged along a circumferential direction of the inner surface of the inner ring. When the smart ring device is worn on a finger, the first electrode and the second electrode simultaneously contact the finger skin.

In some implementations, the first electrode and the second electrode are in a shape of semi-annular or quasi-semi-annular. In this way, the first electrode and the second electrode may enclose to form a closed or generally closed annular configuration, which increases a contact area between the electrodes and the finger skin, thereby facilitating the improvement of detection efficiency and accuracy.

In some implementations, at least one of the first electrode or the second electrode extends over more than two-thirds of a width of the inner ring.

In some implementations, at least one of the first electrode or the second electrode occupies more than two-thirds of the surface area on the inner surface of the inner ring.

In some implementations, the inner ring includes the first electrode, the second electrode and an isolation component between the first electrode and the second electrode along the circumferential direction. The isolation component is made from an electrically insulating material such as a non-metallic material.

In an example, the inner ring may include an inner support structure, as well as the first electrode and the second electrode disposed on the inner surface of the inner support structure. The inner surface of the inner support structure is configured for contacting the finger skin. The inner support structure may be electrically insulating. The first electrode and the second electrode are spaced apart. The isolation component may be made from a material same as or different from that of the inner support structure.

In an example, the outer ring is made from an electrically conductive material such as metal, and the inner ring is made from an electrically insulating material.

In an example, an isolation ring is disposed between the outer ring and the inner ring, and the isolation ring and the outer ring enclose the accommodation space. The isolation ring is made from an electrically insulating material such as a non-metallic material.

In some implementations, a first aperture and a second aperture are provided along the circumferential direction of on the inner ring, where the first electrode protrudes from the inner surface of the inner ring through the first aperture, and the second electrode protrudes from the inner surface of the inner ring through the second aperture.

In some implementations, the smart ring device further includes a circuit board disposed within the accommodation space. The circuit board includes at least two rigid circuit boards and a flexible circuit board for connecting the at least two rigid circuit boards, and the at least one electronic component is disposed on the at least two rigid circuit boards.

Optionally, a part of the components in the smart ring device may be disposed on the rigid circuit boards, and another part may be disposed on the flexible circuit board. Alternatively, all of the components in the smart ring device may be disposed on the one or more rigid circuit boards, and the one or more flexible circuit board is configured for connecting adjacent rigid circuit boards, without any components other than a connection terminal or other types of interconnection components provided thereon. In an example, the electronic components included in the smart ring device, other than the connection components, may be all disposed on the one or more rigid circuit boards.

In some implementations, an inner surface of the outer ring includes at least one of a first mounting region or a second mounting region. At least a portion of the at least two rigid circuit boards is secured to the first mounting region. A locating component is disposed on the second mounting region, and a corresponding mating component adapted to engage the locating component is disposed on the flexible circuit board.

In an example, at least a portion of the one or more rigid circuit boards is adhered or otherwise secured to the first mounting region, and a corresponding mating component adapted to engage the locating component is disposed on the flexible circuit board.

In another example, at least a portion of the flexible circuit board is adhered or otherwise secured to the first mounting region, and a corresponding mating component adapted to engage the locating component is disposed on the one or more rigid circuit boards.

In some implementations, a locating groove is provided on the second mounting region, one end of the locating component is fixed in the locating groove, and the other end of the locating component is connected to the first electrode or the second electrode through the mating component.

In some implementations, the locating component includes a locating post, and the mating component includes a locating aperture.

In an example, the locating post may be connected to the first electrode or the second electrode after passing through the locating aperture, such as by abutting, gluing, welding or the like.

In some implementations, the smart ring device further includes a locating component and a circuit board. The circuit board includes a locating aperture adapted to the locating component, one end of the locating component is connected to the outer ring, and the other end of the locating component passes through the locating aperture on the circuit board 70 and connects to the locating groove 311 on the first electrode 30 or the second electrode 40.

In an example, the first electrode or the second electrode may include a support portion and an electrode portion, where the support portion includes at least two support rods spaced from each other, and the locating post may extend between adjacent support rods.

In some implementations, the at least one electronic component includes an electrodermal activity (EDA) sensing module electrically connected to the first electrode and the second electrode.

The at least one electronic component further includes at least one of a body composition sensing module, a charging module, or a temperature sensing module. The at least one of the body composition sensing module, the charging module, or the temperature sensing module shares at least one of the first electrode or the second electrode with the electrodermal activity sensing module.

The electrodermal activity sensing module may be configured to utilize the first electrode and the second electrode to obtain one or more electrodermal activity signals of a user wearing the smart ring device, and the one or more electrodermal activity signals may be used for analyzing an emotional and/or psychological state of the user.

In an example, the smart ring device may further include at least one processing module, which may be configured to process the one or more electrodermal activity signals to obtain a monitoring result of the emotional and/or psychological state of the user. In another example, the smart ring device may further include a wireless communication module, which may be configured to transmit the one or more electrodermal activity signals obtained by the electrodermal activity sensing module, either raw data or after one or more types of preprocessing is performed, to another electronic device for processing.

In some implementations, the smart ring device further includes an optical detection module disposed within the accommodation space. The optical detection module includes a plurality of optical components and a plurality of convex lenses positioned in correspondence with the optical components. A plurality of openings are provided on the inner surface of the inner ring, and the plurality of convex lenses protrude from the inner ring through the plurality of openings.

In an example, the plurality of convex lenses may form a plurality of convex protrusions, such as racetrack-shaped convex protrusions.

In some implementations, the smart ring device further includes an optical detection module disposed within the accommodation space, and the first electrode and the second electrode are symmetrically arranged on opposite sides of the optical detection module.

In some implementations, the optical detection module includes at least one light emitter and at least one light detector spaced apart from each other along a circumferential direction of the annular housing.

In an example, the optical detection module includes a plurality of light emitters and a light detector, where the plurality of light emitters symmetrically disposed around the light detector along the circumferential direction of the annular housing. Alternatively, the optical detection module includes a light emitter and a plurality of light detectors, where the plurality of light detectors symmetrically disposed around the light emitter along the circumferential direction of the annular housing. Alternatively, the optical detection module includes a plurality of light emitters and a plurality of light detectors, where the plurality of light emitters and the plurality of light detectors are arranged in an alternating pattern along the circumferential direction of the annular housing.

In an example, the at least one light emitter and the at least one light detector form a plurality of optical paths, where a first optical path and a second optical path among the plurality of optical paths have different optical transmission distances.

In some implementations, the smart ring device further includes an optical detection module disposed within the accommodation space. The optical detection module includes a light emitter, as well as a first light detector and a second light detector symmetrically disposed on opposite sides of the light emitter. The light emitter is located in a region where the isolation component is connected to the first electrode and the second electrode.

In some implementations, the smart ring device further includes a circuit board and a curved battery assembly. The circuit board and the curved battery assembly are disposed in different regions of the accommodation space along the circumferential direction of the outer ring.

The circuit board is a curved circuit board. The curved circuit board and the curved battery assembly may enclose to form a ring-like or a generally annular configuration, and a gap between the curved circuit board and the curved battery assembly may be offset from a gap between the first electrode and the second electrode. In an example, the gap between the curved circuit board and the curved battery assembly and the gap between the first electrode and the second electrode may be uniformly or generally uniformly distributed along the circumferential direction of the annular housing. For example, the gap between the circuit board and the curved battery assembly and the gap between the first electrode and the second electrode divide the circumference of the annular housing into four generally equal segments.

The smart ring device provided in the present disclosure has an annular housing including an outer ring, an inner ring, and an accommodation space therebetween, at least one electronic component is disposed within the accommodation space, and the first and second electrodes electrically connected to the at least one electronic component are spaced apart along the circumferential direction of the inner surface of the annular housing, enabling the monitoring of physiological parameters of the user by using the first and second electrodes.

### BRIEF DESCRIPTION OF DRAWINGS

The accompanying drawings are incorporated into and constitute a part of the specification, illustrating embodiments consistent with the present disclosure, and are used in conjunction with the specification to explain the principles and implementations of the present disclosure.
FIG. 1 is a schematic structural diagram of a smart ring device according to some embodiments.
FIG. 2 is a disassembled schematic diagram of the smart ring device according to some embodiments.
FIG. 3 is another schematic structural diagram of the smart ring device according to some embodiments.
FIG. 4 is a schematic structural diagram of an outer ring of the smart ring device according to some embodiments.
FIG. 5 is another schematic structural diagram of the smart ring device according to some embodiments.
FIG. 6 is a schematic block diagram of the smart ring device according to some embodiments.
FIG. 7 is a schematic block diagram of charging the smart ring device according to some embodiments.
FIG. 8 is a schematic diagram of charging the smart ring device according to some embodiments.
FIG. 9 is a schematic diagram of manufacturing the smart ring device according to some embodiments.
FIG. 10 is another schematic diagram of manufacturing the smart ring device according to some embodiments.
FIG. 11 is another schematic diagram of manufacturing the smart ring device according to some embodiments.

### Description of reference numerals:

100: smart ring device, 10: annular housing, 11: outer ring, 111: inner surface of the outer ring, 112: first mounting region, 113: second mounting region, 1131: locating component, 1132: groove, 12: inner ring, 121: first aperture, 122: second aperture, 123: opening, 13: accommodation space, 20: electronic device, 21: electrodermal activity (EDA) sensing module, 22: charging module, 23: temperature sensing module, 24: optical detection module, 241: convex lens, 242: first optical component, 243: second optical component, 2431: first light emitter, 2432: second light emitter, 244: third optical component, 30: first electrode, 31: support portion, 311: locating groove, 32: detection portion, 40: second electrode, 50: isolation component, 60: isolation ring, 70: circuit board, 71: rigid circuit board, 72: flexible circuit board, 721: mating component, 73: wire, 80: battery assembly, 200: charging case, 201: charging terminal, 300: upper positioning jig, 400: lower positioning jig, 500: silicone jig.

### DETAILED DESCRIPTION

Example embodiments will be described herein in detail with reference to the accompanying drawings. In the description referring to the drawings, a same numeral in different drawings denote a same or similar elements unless otherwise specified. The example embodiments described in the following do not represent all embodiments consistent with the present disclosure. Instead, they are merely examples of the methods and devices consistent with some aspects of the present disclosure as detailed in the appended claims.

The terminology used in the present disclosure is used for the purpose of describing particular embodiments and is not intended to limit the present disclosure. Unless otherwise defined, technical or scientific terms used herein shall have the ordinary meaning as understood by one of ordinary skill in the art to which the present disclosure belongs. The terms "first", "second" and the like are used in the specification and claims of the present disclosure do not denote any order, quantity, or importance, but are used merely to distinguish different components. Similarly, terms such as "a" or "an" do not denote a quantity limitation, but rather denote the presence of at least one. The term "multiple" or "a plurality of" denotes two or more. The terms such as "comprises", "includes" and the like represent that an element or object preceding the terms encompass the elements or objects as well as their equivalents after the terms, without excluding other elements or objects. The terms "connected", "coupled" and the like are not limited to physical or mechanical connections, and may include electrical connections, which may be direct or indirect. The singular forms "a", "an", and "the" used in the specification and appended claims of the present disclosure are also intended to include the plural forms, unless the context clearly indicates otherwise. It should be understood that the term "and/or" used herein refers to and includes any or all possible combinations of one or more of the associated listed items. The term "fitting" or "adapting" in the present disclosure includes, but is not limited to, the installation or removal relationship between elements or structures as well as the mounting or dismounting process.

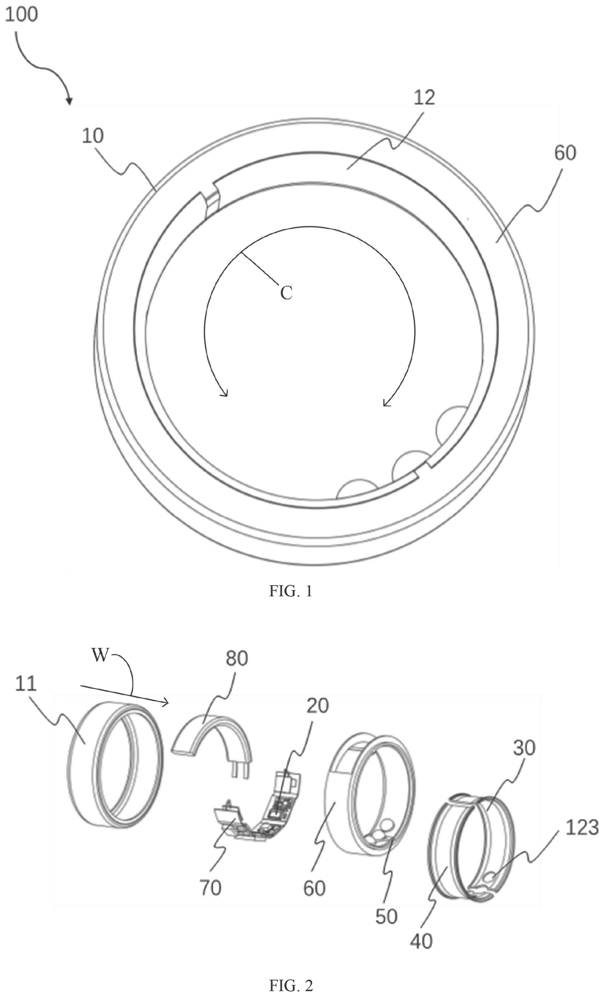

Referring to FIGs. 1 to 5, the present disclosure provides a smart ring device 100, including an annular housing 10 and at least one electronic component 20. The annular housing 10 includes an outer ring 11, an inner ring 12, and an accommodation space 13 located between the outer ring 11 and the inner ring 12. The at least one electronic component 20 is disposed within the accommodation space 13. The smart ring device 100 further includes a first electrode 30 and a second electrode 40. The first electrode 30 and the second electrode 40 are spaced apart from each other along the circumferential direction C(shown in FIG. 1) of the inner surface of the annular housing 10. The first electrode 30 and the second electrode 40 are electrically connected to the at least one electronic component 20.

In the smart ring device 100 provided by the present disclosure, the accommodation space is arranged between the outer ring 11 and the inner ring 12 of the annular housing 10. The at least one electronic component 20 is disposed within the accommodation space. The first electrode 30 and the second electrode 40, which are electrically connected to the at least one electronic component 20, are arranged along a circumferential direction of the inner surface of the annular housing 10 for contacting the finger skin of a user. Thus, when the smart ring device 100 is worn on a user's finger, the first electrode 30 and the second electrode 40 may be configured for contacting the finger skin and obtaining one or more physiological signals from the user. The obtained physiological signals may be transmitted to the electronic component 20, thereby realizing the health monitoring of the user based on the smart ring device 100.

The first electrode 30 and the second electrode 40 are configured to obtain one or more types of physiological signals, including at least one of an electrodermal activity (EDA) signal, a body impedance signal, a heart rate detection signal, a blood pressure signal, an electrocardiogram (ECG) signal, or a body temperature signal or the like.

In some embodiments, at least one of the first electrode 30 or the second electrode 40 is multiplexed to acquire multiple types of physiological signals, thereby reducing the number of electrodes in the smart ring device 100, and further streamlining the structure of the smart ring device and reducing device cost. In an example, at least one of the first electrode 30 or the second electrode 40 is shared to acquire both an EDA signal and a body temperature signal. In another example, the first electrode 30 and the second electrode 40 are shared to acquire both an EDA signal and an ECG signal. In some other embodiments, while the first electrode 30 and the second electrode 40 are configured for acquiring one or more physiological signals, the first electrode 30 and the second electrode 40 are also served as charging terminals. For instance, while the first electrode 30 and/or the second electrode 40 are served as body temperature sensing electrodes, they the first electrode 30 and the second electrode 40 are also served as charging terminals. The specific implementations of sharing the electrodes are not limited herein.

The annular housing 10 includes an outer ring 11 and an inner ring 12. The outer surface of the outer ring 11 may form at least a portion of the outer surface of the annular housing 10, and the inner surface of the inner ring 12 may form at least a portion of the inner surface of the annular housing 10.

The outer ring 11 includes an electrically conductive material such as a metallic material. In some embodiments, the outer ring 11 may be made from one of titanium alloy, a stainless steel, or aluminum alloy, etc. In an example, the outer ring 11 is made from titanium alloy, which provides the smart ring device 100 with a relatively high overall hardness and improved aesthetics.

The inner ring 12 may include an electrically conductive material such as a metallic material, or include an electrically insulating material such as a non-metallic material. Alternatively, a portion of the inner ring 12 is made from an electrically conductive material, such as a metallic material, and another portion is made from an electrically insulating material, such as a non-metallic material.

In some embodiments, the inner ring 12 is made from a non-metallic material, such as a moldable material like epoxy resin. In this case, the inner ring 12 includes at least one aperture, and the first electrode 30 and the second electrode 40 protrude from the inner surface of the inner ring 12 through the at least one aperture. Alternatively, the first electrode 30 and the second electrode 40 include a metallic coating layer on the inner surface of the inner ring 12.

In some other embodiments, the inner ring 12 is made from a metallic material. The metallic material of the inner ring 12 may be the same or different from the metallic material of the outer ring 11. In this case, at least a portion of the inner surface of the inner ring 12 may serve as the first electrode 30 and the second electrode 40. For example, the first electrode 40 may include a first portion of the inner surface of the inner ring 12, and the second electrode includes a second portion of the inner surface of the inner ring 12. Alternatively, at least one aperture is provided on the inner surface of the inner ring 12, and the first electrode 30 and the second electrode 40 protrude from the inner surface of the inner ring 12 through the at least one aperture. An electrically insulating layer is provided between the first electrode 30 and the second electrode 40 and the inner surface of the inner ring 12.

The accommodation space 13 is provided between the outer ring 11 and the inner ring 12. In some embodiments, a circumference wall of the accommodation space 13 may include the outer ring 11 and the inner ring 12. Alternatively, one or more other components, such as one or more other annular components, may be provided between the outer ring 11 and the inner ring 12, and accordingly, the circumference wall of the accommodation space 13 may include at least one of the outer ring 11 or the inner ring 12. In an example, both the outer ring 11 and the inner ring 12 are made from metallic materials, and an isolation ring 60 made from an electrically insulating material such as non-metal (e.g., epoxy resin, polyurethane, polyethylene, etc.) may be provided between the outer ring 11 and the inner ring 12. The isolation ring 60 may be formed from potting or any other suitable process. The accommodation space 13 may be formed between the outer ring 11 and the isolation ring 60, or formed between the inner ring 12 and the isolation ring 60. In an example, the accommodation space 13 may be formed between the outer ring 11 and the isolation ring 60, and the circumference wall of the accommodation space 13 may include at least a portion of the outer ring 11 and at least a portion of the isolation ring 60. The isolation ring 60 is disposed on the outer surface of the inner ring 12. The inner surface of the annular housing 10 includes the surface of the inner ring 12, which is referred to as the first surface, and the outer surface of the inner ring 12 includes the second surface opposite to the first surface. The isolation ring 60 may, for example, include or be formed by a coating or a potted layer on the outer surface of the inner ring 12. In another example, the accommodation space 13 may be arranged between the inner ring 12 and the isolation ring 60, and the circumference wall of the accommodation space 13 may include at least a portion of the inner ring 12 and at least a portion of the isolation ring 60. The isolation ring 60 is disposed on the inner surface of the outer ring 11. The isolation ring 60 may, for example, include or be formed by a coating or a potted layer on the inner surface of the outer ring 11.

The at least one electronic component 20 is disposed within the accommodation space 13. The at least one electronic component 20 may include at least one of a charging assembly or a physiological sensor. The physiological sensor may acquire physiological data based on one or more electrode signals, such as at least one of a bio-electrical impedance analysis (BIA) sensor, a body temperature sensor, an electrocardiogram sensor, or an EDA sensor or the like. Additionally, at least one of a processor, a memory, a battery component, a communication component (e.g., wired and/or wireless communication module), a locating component or the like may be disposed within the accommodation space 13, which is not limited in the present disclosure.

In some embodiments, an optical detection module 24 is disposed within the accommodation space 13. The optical detection module 24 may be configured to detect a pulse signal from the user to obtain one or more physiological parameters such as heart rate, blood oxygen saturation, blood pressure, blood glucose or the like. Alternatively, the smart ring device may be further configured to determine whether the one or more physiological parameters are abnormal and promptly alert the user when an abnormal condition is detected.

The optical detection module 24 may include a plurality of optical components, such as at least one light emitter and at least one light detector. The light emitter may include one or more LEDs for emitting optical signals of at least one wavelength. The one or more LEDs may include an LED associated with a single wavelength, such as, green light, red light, or infrared light or the like, an LED associated with dual wavelengths, such as red light and infrared light, or an LED associated with three wavelengths, such as green light, red light, and infrared light. The at least one light detector may include a photodiode (PD) configured to detect one or more optical signals within a certain wavelength range, such as a wavelength range from 400 nm to 1000 nm. The optical detection module 24 may be a reflective or transmissive optical sensor. A distance between the light emitter and the light detector may be in the range from 3 mm to 12 mm, which is not limited herein. The light emitter may be configured to emit optical signals of one or more wavelengths. The emitted optical signals may be received by the light detector after being incident on the finger skin and reflected and/or refracted by the finger skin. After the optical signals received by the light detector are analyzed and processed, a physiological measurement may be obtained. For instance, the optical detection module 24 may utilize green light for heart rate detection. For another instance, the optical detection module 24 may utilize red and infrared light for blood oxygen saturation detection. The light emitter and the light detector may be located on a same side relative to the finger skin. For instance, the optical emitter and the optical detector may be disposed adjacent to each other within the annular housing 10. Alternatively, the light emitter and the light detector may be located on different sides relative to the finger skin. For instance, the light emitter and the light detector may be located in opposite regions of the annular housing 10 of the smart ring device 100. The present disclosure does not set limitations on the implementations of the optical detection module 24.

In some embodiments, to enable the optical signals emitted from the light emitter to be incident on the finger skin and to allow the optical signals returning from the finger skin to be received by the light detector, one or more light-transmitting regions may be provided on the inner ring 12 in correspondence with the at least one light emitter and the at least one light detector, and a light-blocking region is provided between the light-transmitting regions. In an example, the inner ring 12 may be made from a light-transmitting material, such as glass, sapphire, epoxy resin, transparent plastic or the like, and a light-blocking layer may be provided at locations not corresponding to the at least one light emitter and the at least one light detector, so as to block the direct light transmission passage of light between the light emitter and the light detector and improve aesthetics of the smart ring device. For instance, an ink layer may be provided on the surface of the inner ring. The ink layer includes ink covers areas not corresponding to optical components, or include ink covers regions between regions corresponding to optical components. Alternatively, a bracket may be provided between the inner ring 12 and the optical detection module 24, and the bracket includes corresponding light-transmitting and light-shielding regions. In another example, the inner ring 12 may be made from an opaque material, and at least one aperture is provided at positions corresponding to the at least one light emitter and the at least one light detector, with at least one light-transmitting lens disposed within the at least one aperture to form one or more light-transmitting regions.

In some embodiments, the optical detection module 24 may further include at least one convex lens 241 corresponding to the plurality of optical components. The convex lens 241 may be elliptical or any other shapes to converge optical signals, which in turn is conductive to reduce the power consumption of the optical detection module 24. Additionally, the convex lens 241 may serve as a part of the inner surface of the smart ring device 100, enhancing contact tightness with finger skin to improve measurement accuracy. The convex lens 241 may also be configured as a positional guide during wearing or charging of the smart ring device 100. For example, when the smart ring device 100 is placed in the charging case 200, the convex lens 241 may align with guide grooves on the charging case 200, thus achieving the effect of positioning and anti-misalignment, and improving charging safety. In the example shown in FIG. 2, the convex lens has a shape of an elliptical racetrack, and is formed from a light-transmitting material, such as transparent silicone or epoxy resin, or the like.

In an example, at least one convex lens 241 may be provided on the inner surface of the annular housing 10 at positions corresponding to the optical components. The inner ring 12 may include least one opening 123, and the at least one convex lens 241 is arranged within the at least one opening 123 and protrudes from the inner surface of the inner ring 12. For instance, at least one convex lens 241 is disposed in the accommodation space 13, and an opening 123 is provided at a corresponding position of the inner ring 12 to allow the convex lens 241 to protrude from the opening 123. In another example, the inner ring 12 may be made from light-transmitting material and have at least one protrusion at positions corresponding to the optical components, and the at least one protrusion may be configured as the convex lens 241. In other words, the inner surface of the inner ring 12 may include at least one protrusion for converging optical signals.

In some embodiments, to prevent light signals emitted by the light emitter from directly entering the light detector and causing crosstalk, the optical detection module 24 may further include an optical barrier disposed between the light emitter and the light detector. For instance, an end of the optical barrier is connected to the circuit board on which the optical components are provided, and the other end of the optical barrier is connected to the surface of the inner ring 12, but no limitation is set on the specific implementations of the optical barrier.

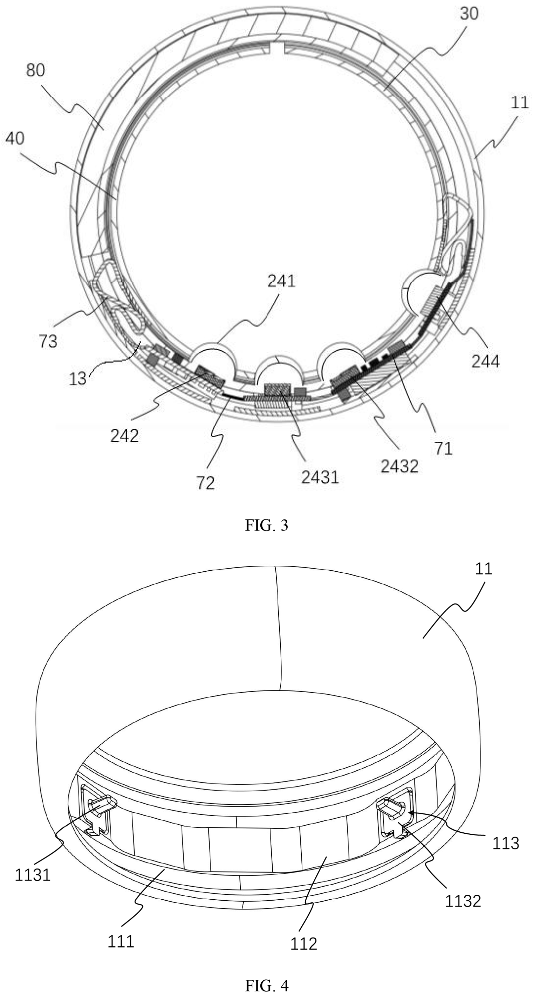

In some optional embodiments, referring to FIGs. 2 and 3, both the first electrode 30 and the second electrode 40 are in the shape of semi-annular or approximately semi-annular. The first electrode 30 and the second electrode 40 may enclose to form an annular or approximately annular configuration along the circumferential direction of the inner surface of the inner ring 12. In this case, the first electrode 30 and the second electrode 40 may have a relatively large surface area, and correspondingly, have a relatively large contact area with the finger skin, which is beneficial for improving the efficiency and accuracy of physiological detection. In an example, the first electrode 30 and the second electrode 40 collectively extend over more than two-thirds of the circumference of the inner ring 12. In another example, at least one of the first electrode 30 or the second electrode 40 occupies a dimensional ratio exceeding two-thirds along the width direction (*W*, as shown in FIG. 2) of the inner ring 12. In another example, the first electrode 30 and the second electrode 40 occupy more than two-thirds of the surface area on the inner surface of the inner ring 12. In this case, the first electrode 30 and the second electrode 40 have a relatively large contact area with the finger skin of the user.

In some embodiments, referring to FIG. 2, the inner ring 12 includes the first electrode 30, the second electrode 40, and an isolation component 50 disposed between the first electrode 30 and the second electrode 40 along the circumferential direction of the inner ring 12. The isolation component 50 is made from an electrically insulating material such as a non-metallic material. This configuration ensures that a contact area between both the first electrode 30 and the second electrode 40 and the finger skin is sufficiently large, which is beneficial for the smart ring device 100 to accurately and efficiently obtain signals through the first electrode 30 and the second electrode 40. Meanwhile, the isolation component 50 provides electrical isolation between the first electrode 30 and the second electrode 40, and also provides an antenna clearance area. In the example shown in FIG. 2, two isolation components 50 are provided in the gap between the first electrode 30 and the second electrode 40, located at the connection portions of the first electrode 30 and the second electrode 40, respectively. However, in some other embodiments, the number of isolation components 50 may be more or less, which is not limited in the present disclosure.

In some examples, the first electrode 30 and the second electrode 40 may be identical, which ensures signal acquisition performance and facilitates manufacturing and processing.

Furthermore, in some embodiments, at least one of the first electrode 30 or the second electrode 40 includes at least one opening 123. The at least one opening may be provided corresponding to the plurality of optical components included in the optical detection module 24 disposed within the accommodation space 13, allowing the plurality of convex lenses 241 corresponding to the plurality of optical components to protrude through the at least one opening 123.

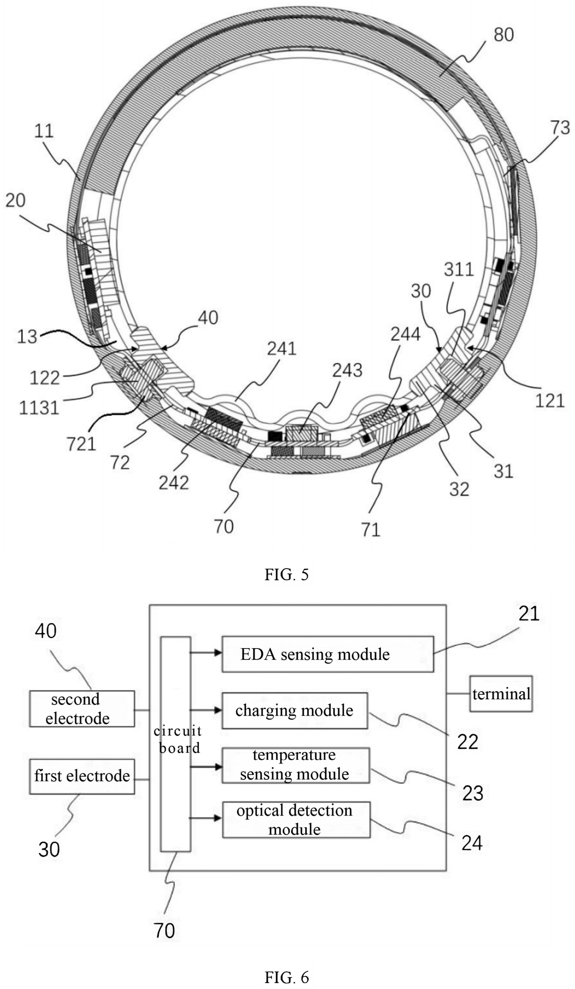

In the examples shown in FIGs. 2 and 5, three optical components are disposed within the accommodation space 13 along the circumferential direction of the annular housing 10, namely a first optical component 242, a second optical component 243, and a third optical component 244. The second optical component 243 is located in a region where the isolation component 50 is connected to the first electrode 30 and the second electrode 40. The first optical component 242 and the third optical component 244 are disposed on opposite sides of the second optical component 243. In an example, the first optical component 242 and the third optical component 244 are symmetrically arranged with respect to the second optical component 243. Each of the first optical component 242, the second optical component 243, and the third optical component 244 may be a light emitter or a light detector. In some examples, the second optical component 243 is a light emitter, and the first optical component 242 and the third optical component 244 both are light detectors. In some other examples, the second optical component 243 is a light detector, and the first optical component 242 and the third optical component 244 both are light emitters. In some other examples, the number of optical components may be more or less, and the optical components may be arranged in other configurations, which is not limited in the present disclosure. An example is described herein, where the second optical component 243 is a light emitter, and both the first optical component 242 and the third optical component 244 are light detectors. The second optical component 243 may form a first optical detection channel with the first optical component 242, and form a second optical detection channel with the third optical component 244. In some examples, in the case where the second optical component 243 is an LED for emitting light signals associated with two wavelengths or an LED for emitting light signals associated with three wavelengths, the second optical component 243 may include more than two light-emitting elements, each of which may form different optical detection channels with the first optical component 242 and/or the third optical component 244, which is not limited in the present disclosure.

In the example shown in FIG. 3, the optical detection module 24 includes four optical components: a first light emitter 2431, a second light emitter 2432, a first light detector 242, and a second light detector 244. The first light emitter 2431 and the second light emitter 2432 are disposed near the region where the isolation component 50 connects the first electrode 30 and the second electrode 40. The first light detector 242 and the second light detector 244 are disposed on two sides of the first light emitter 2431 and the second light emitter 2432. The first light emitter 2431 and the second light emitter 2432 are configured to emit visible light or infrared light, and together with the first light detector 242 and the second light detector 244, forming at least one optical detection channel for obtaining physiological signals. In an example, a distance between the first light emitter 2431 and the first light detector 242 is 4 mm, forming a first optical detection channel. A distance between the first light emitter 2431 and the second light detector 244 is 12 mm, forming a second optical detection channel. A distance between the second light emitter 2432 and the first light detector 242 is 8 mm, forming a third optical detection channel. A distance between the second light emitter 2432 and the second light detector 244 is 8 mm, forming a fourth optical detection channel. The plurality of optical components form four optical detection channels, enabling a relatively high measurement signal quality and accurate physiological parameters. Similarly, in some examples, in the case where the first light emitter 2431 or the second light emitter 2432 is an LED for emitting light signals associated with two wavelengths or an LED for emitting light signals associated with three wavelengths, more optical detection channels may be formed, or each optical detection channel may include more optical components, which is not limited in the present disclosure.

Furthermore, a convex lens 241 is correspondingly provided for each optical component. In the example shown in FIG. 2, the first electrode 30 and the second electrode 40 each include a full or complete opening 123 in the middle region and a half-opening at an end. The half-opening of the first electrode 30 and the half-opening of the second electrode 40 may form a full or complete opening 123 after assembly of the smart ring device 100. In this case, the two convex lenses 241 corresponding to the first optical component 242 and the third optical component 244 protrude through the complete openings 123 provided on the first electrode 30 and the second electrode 40, respectively, and the convex lens 241 corresponding to the second optical component 243 protrudes through the opening 123 formed by combination of the first electrode 30 and the second electrode 40.

Further, in the example shown in FIG. 2, an isolation ring 60 is provided between the outer ring 11 and the inner ring 12, and the accommodation space 13 is formed between the isolation ring 60 and the outer ring 11. The isolation ring 60 may be made from a non-metallic material such as silicone or resin, and at least one of the convex lens 241 or the isolation component 50 may be disposed on the isolation ring 60. The outer ring 11 and the inner ring 12 are respectively secured to opposite sides (i.e., the inner side and the outer side) of the isolation ring 60. This arrangement allows the isolation ring 60 to provides an electrically insulating effect and improve the overall aesthetics of the smart ring device 100. The accommodation space 13 provides a receiving space for the one or more electronic components 20, enabling rational utilization of the space of the smart ring device 100 and realization of various functions.

In some other embodiments, as shown in FIG. 5, a first aperture 121 and a second aperture 122 are symmetrically provided along the circumferential direction of the inner surface of the inner ring 12. The first electrode 30 protrudes from the inner surface of the inner ring 12 through the first aperture 121, and the second electrode 40 protrudes from the inner surface of the inner ring 12 through the second aperture 122. In this case, the inner surface of the inner ring 12 may be made from a non-metallic material, or, an electrically insulating layer may be provided between the inner surface of the inner ring 12 and the first and second electrodes 40. The first electrode 30 and the second electrode 40 are disposed on opposite sides of the optical detection module 24, e.g., surrounding the optical components 242, 243, and 244 along the circumferential direction. In this case, a plurality of convex lenses 214 may be disposed on the inner surface of the inner ring 12. For example, the inner surface of the inner ring 12 includes a plurality of protrusions configured as the convex lenses 214, but the present disclosure is not limited thereto.

In some examples, the smart ring device 100 further includes a circuit board 70 disposed within the accommodation space 13. The one or more electronic components 20 are disposed on the circuit board 70. Alternatively, one or more other components, such as at least one of the optical detection module 24, a temperature sensing module 23, a charging module 22, an electrodermal activity sensing module 21, or a communication module, may be disposed on the circuit board 70. An end of each of the first electrode 30 and the second electrode 40 may be disposed on the circuit board 70, and the other end of each of the first electrode 30 and the second electrode 40 protrudes from the inner surface of the inner ring 12. In this case, both the first electrode 30 and the second electrode 40 may have a certain thickness. For instance, as shown in FIG. 5, the first electrode 30 and the second electrode 40 may include a support portion 31 and a detection portion 32. The support portion 31 is arranged on the circuit board 70, and the detection portion 32 protrudes from the inner surface of the inner ring 12. The support portion 31 may be secured to the circuit board 70, for example, via adhesive, locating pins, screws, etc. Alternatively, the support portion 31 may be secured to the outer ring 11. For instance, as shown in FIG. 5, the support portion 31 includes a locating groove 311, and a locating component 1131 provided on the outer ring 11 is disposed in the locating groove 311, enabling positioning of the first electrode 30 and the second electrode 40. To make the positions of the first electrode 30 and the second electrode 40 more stable, in some examples, adhesive or other fastening components may be provided at the contact region between the locating component 1131 and the detection portion 32, which is not limited herein.

As shown in FIGs. 3 and 5, to adapt to the annular structure of the smart ring device 100 and mount as many electronic components 20 as possible on the circuit board 70, the circuit board 70 may be configured in a curved shape. The circuit board 70 may include one or more rigid circuit boards 71 and one or more flexible circuit boards 72. For instance, the circuit board 70 includes at least two rigid circuit boards 71 and one or more flexible circuit boards 72 connecting the at least two rigid circuit boards 71. The rigid circuit board 71 may be a printed circuit board (PCB). The one or more electronic components 20 may be disposed on the at least two rigid circuit boards 71 to ensure their stability. The one or more flexible circuit boards 72 may be a flexible printed circuit (FPC), and the one or more flexible circuit boards 72 connect adjacent ones of the at least two rigid circuit boards 71 to form a curvature adapted to the annular configuration of the smart ring device 100. In some embodiments, the curvature of the flexible circuit board 72 may be adapted to the accommodation space of the smart ring device 100, enabling a same circuit board 70 to be adapted to smart ring devices 100 with different sizes and configurations, thereby achieving the versatility of the circuit board 70. The circuit board 70 may be a rigid-flex circuit board. By adjusting the curvature of the one or more flexible circuit boards 72 and the angle between adjacent rigid circuit boards 71 according to the shape and size of the smart ring device 100, the curvature and overall length of the rigid-flex circuit board may be changed according to the size and shape of the smart ring device 100 without requiring additional mechanisms and parts. In some examples, a reinforcing plate such as a steel sheet may be provided at the flexible circuit board 72 to enhance the structural strength of the flexible circuit board 72, which is not limited in the present disclosure.

In some embodiments, the at least one electronic component 20 in the smart ring device 100 may all be disposed on the at least two rigid circuit boards 71, and the flexible circuit board 72 is free of any electronic component 20, and provided only with some necessary connection terminals or connectors. In some other embodiments, some of the at least one electronic component 20 in the smart ring device 100 may be disposed on one or more rigid circuit boards 71, and the others of the at least one electronic component 20 may be disposed on one or more flexible circuit boards 72. In some other embodiments, one or more additional electronic components of the smart ring device 100 may be disposed on the rigid circuit board 71 or the flexible circuit board 72. For example, the at least one electronic component 20 is disposed on the rigid circuit board 71, and one or more additional electronic components are disposed on the flexible circuit board 72, but the present disclosure is not limited thereto.

The first electrode 30 and the second electrode 40 may be electrically connected to the rigid circuit board 71 and/or the flexible circuit board 72 of the circuit board 70. In some examples, the circuit board 70 may be electrically connected to the first electrode 30 and the second electrode 40 through a conductive wire 73 or other types of electrical connection mechanisms. The first electrode 30 and the second electrode 40 may be electrically connected to the rigid circuit board 71 or the flexible circuit board 72. In some embodiments, a bonding pad may be mounted on the rigid circuit board 71 or the flexible circuit board 72, and the first electrode 30 and the second electrode 40 are soldered to the bonding pad by surface mount technology (SMT). For instance, one end of the conductive wire 73 may be soldered to the first electrode 30 and the second electrode 40, and the other end of the conductive wire 73 is connected to the rigid circuit board 71 or the flexible circuit board 72. In some embodiments, a metal sheet may be disposed within the accommodation space 13 of the annular housing 10, and connections between the outer ring 11, the inner ring 12, or the circuit board 70 and the first electrode 30 and the second electrode 40 are achieved by soldering to the metal sheet. Alternatively, conductive adhesive may be used to bond the circuit board 70 to the first electrode 30 and the second electrode 40, achieving electrical connection therebetween.

The optical detection module 24 may be disposed on the rigid circuit board 71 or the flexible circuit board 72. In some embodiments, a light-impermeable material may be provided in the region of the circuit board 70 for disposing optical components. For instance, the region of the circuit board 70 for mounting the optical components may be made from light-impermeable material or be provided with a light-shielding layer to avoid light crosstalk between different optical components. The region of the circuit board 70 between the light emitter and the light detector may be provided with a light-impermeable material. In some other embodiments, the first optical component 242 and the third optical component 244 may be encapsulated on the rigid circuit board 71 with a light-transmitting material, such as silicone or resin, and an opaque optical barrier may be disposed around the second optical component 243. Alternatively, an opaque optical barrier may be disposed around the first optical component 242 and the third optical component 244, respectively, which is not limited in the present disclosure.

The circuit board 70 may be secured within the accommodation space 13 of the smart ring device 100 by a variety of positioning structures, such as adhesive, locating components, welding or the like. In some embodiments, as shown in FIG. 4, at least one of a first mounting region 112 or a second mounting region 113 is provided on the inner surface 111 of the outer ring. At least a portion of the rigid circuit board 71 is secured to the first mounting region 112 by a first locating structure, and at least a portion of the flexible circuit board 72 is secured to the second mounting region 113 by a second locating structure different from the first locating structure. In some examples, the first locating structure may include adhesive or welding, and the second locating structure may include a locating component. For instance, the second mounting region 113 includes a locating component 1131, and the flexible circuit board 72 includes a mating component 721 engaging the locating component 1131. The locating component 1131 may include a locating post, and the mating component 721 may include a locating aperture. Alternatively, the locating component may include a screw, and the mating component 721 may include a nut, etc. In this way, the rigid circuit board 71 and the flexible circuit board 72 may be secured in the accommodation space 13 respectively by different locating structures, improving the positioning effectiveness of the circuit board 70.

By adapting and connecting the mating component 721 on the flexible circuit board 72 with the locating component 1131, the flexible circuit board 72 may be secured to the outer ring 11, facilitating fastening the circuit board 70 to the outer ring 11, preventing the circuit board 70 from moving relative to the outer ring 11, ensuring the functional stability of the smart ring device 100, and improving the assembly convenience of the smart ring device 100.

In some embodiments, to further improve the positioning effect, a groove 1132 is provided on the second mounting region 113, as shown in FIG. 4 and FIG. 5. One end of the locating component 1131 is fixedly disposed within the groove 1132, and the other end of the locating component 1131 is connected to the first electrode 30 or the second electrode 40 through the mating component 721. Thus, the locating component 1131 not only serves to position the circuit board 70, but also further assist in positioning of the electrode. The locating component 1131 may be secured within the groove 1132 by adhesive dispensing or snap-fit engagement or the like, and connected to the first electrode 30 or the second electrode 40 by adhesive dispensing, soldering, or snap-fit engagement or the like. Correspondingly, the first electrode 30 and/or the second electrode 40 may be provided with a locating groove 311, and an end of the locating component 1131 may be disposed within the locating groove 311 and secured thereto by means such as adhesive dispensing, snap-fit engagement, etc.

As shown in the example of FIG. 5, the outer ring 11 of the smart ring device 100 includes a locating component 1131, and the circuit board 70 includes a locating aperture adapted to the locating component 1131. One end of the locating component 1131 is fixedly secured to the outer ring 11, and the other end of the locating component 1131 passes through the locating aperture on the circuit board 70 and connects to the locating groove 311 on the first electrode 30 or the second electrode 40. This configuration allows simultaneous fixation of both the circuit board 70 and the electrode through the adaptive locating structure including the locating component and the locating aperture.

In some embodiments, as shown in FIGs. 3 and 5, the smart ring device 100 further includes a curved battery assembly 80, which is semi-annular or generally semi-annular in shape. The curved battery assembly 80 and the circuit board 70 are disposed in different areas along the circumferential direction in the accommodation space 13. For example, the curved battery assembly 80 and the circuit board 70 may enclose a shape adapted to the annular housing 10, such as an annular or substantially annular shape. In some examples, there may be at least one first gap between the curved battery assembly 80 and the circuit board 70. For instance, the curved battery assembly 80 and the circuit board 70 are completely non-overlapping. In the examples shown in FIGs. 2 and 3, the curved battery assembly 80 and the circuit board 70 may be arranged in a displaced manner relative to the first electrode 30 and the second electrode 40, such that the first gap between the curved battery assembly 80 and the circuit board 70 does not overlap with the second gap between the first electrode 30 and the second electrode 40 along the circumferential direction of the annular housing 10. This configuration allows more efficient utilization of the annular accommodation space 13, improves the compactness of the internal structure of the smart ring device 100, and reduces the thickness of the smart ring device 100. In some examples, the at least one first gap between the curved battery assembly 80 and the circuit board 70 and the at least one second gap between the first electrode 30 and the second electrode 40 are uniformly distributed along the circumferential direction of the annular housing 10. For instance, as shown in FIG. 2, the curved battery assembly 80 and the circuit board 70 are spaced apart vertically, i.e., the battery assembly 80 and the circuit board 70 are arranged in the upper and lower portions of the annular housing 10. The first electrode 30 and the second electrode 40 are spaced apart horizontally, i.e., the first electrode 30 and the second electrode 30 are arranged in the left and right portions of the annular housing 10. Therefore, a line connecting the two first gaps and a line connecting the two second gaps are perpendicular or approximately perpendicular to each other, but the embodiments of the present disclosure are not limited thereto.

In some embodiments, different components of a same module may be disposed on a same circuit board, or different components of the same module may be disposed on different circuit boards. For example, the first electrode 30 and the second electrode 40 may be disposed on different circuit boards. For another example, the light emitter and the light detector of the optical detection module 24 may be disposed on different circuit boards, or different light emitters and/or different light detectors of the optical detection module 24 may be disposed on different circuit boards. In an example, the one or more light emitters of the optical detection module 24 may be disposed on a same circuit board, while different light detectors may be disposed on different circuit boards, which are different from the circuit board to which the light emitters are mounted. In another example, each individual optical component of the optical detection module 24 may be disposed on a separate circuit board, e.g., each LED for emitting light signals associated with two or three wavelengths is disposed on a separate circuit board, and each PD is disposed on a separate circuit board, which is not limited in the present disclosure.

In some embodiments, the at least one electronic component 20 may include an electrode-based charging module or one or more physiological sensors. As shown in the example of FIG. 6, the at least one electronic component 20 may include at least one of an electrodermal activity (EDA) sensing module 21 or a body composition sensing module, which is electrically connected to the first electrode 30 and the second electrode 40. Additionally, the accommodation space 13 may further include at least one of a charging module 22 or a temperature sensing module 23. Optionally, at least one of the charging module 22 or the temperature sensing module 23 may share the first electrode 30 and the second electrode 40 with at least one of the EDA sensing module 21 or the body composition sensing module. In this case, when the smart ring device 100 is worn on a finger, the first electrode 30 and the second electrode 40 contact the finger skin to obtain signals and transmit the obtained signals to one or more sensing modules disposed on the circuit board 70. The one or more sensing modules may be configured to process the signals to obtain physiological detection data. In some embodiments, the smart ring device 100 may further include a processing module such as a controller and a switching circuit. The switching circuit is configured to switch a connection state between the first electrode 30 and the second electrode 40 and one or more modules, enabling the first electrode 30 and the second electrode 40 to measure different types of physiological data, or to switch between charging and physiological measurement. The controller is configured to send control signals to the switching circuit. In some other embodiments, instead of including a controller, the smart ring device 100 may include a wireless communication module, such as Bluetooth, WIFI, etc. The wireless communication module may be configured to receive control signals sent by a mobile terminal device or a server device, and the switching circuit may be configured to switch the connection status between the first electrode 30 and the second electrode 40 and one or more sensing modules under the control of the control signals. Additionally, after obtaining the physiological signals and/or processed data, the smart ring device 100 may send the physiological signals and/or processed data to the mobile terminal device or the server device through the wireless communication module, so that the mobile terminal device or the server device performs further processing on the physiological signal and/or processed data to obtain the physiological detection result, and/or, output the received processed data and/or physiological detection result to the user. The mobile terminal device may include an electronic device such as a mobile phone, another wearable device such as a smart bracelet, a tablet computer, a vehicle-mounted terminal or the like. The mobile terminal device may be configured to output the physiological detection result via audio, haptic, a display, etc., so that the user can view his or her health data on the mobile terminal, such as emotional state information, physiological stress information, electrocardiogram information, body composition information, body temperature, heart rate, blood oxygen saturation, etc. Alternatively, the mobile terminal device or the server device may be configured to obtain a health improvement suggestion based on the physiological signal and/or processed data and output the health improvement suggestion to the user. Optionally, the user may interact with the mobile terminal device to instruct the smart ring device 100 to perform specific operations or functions.

In some embodiments, the EDA sensing module 21 may be configured to detect electrodermal activity signals indicating physiological and emotional changes of the user through the first electrode 30 and the second electrode 40. The EDA sensing module 21 may be configured to transmit the electrodermal activity signals to the mobile terminal device or the server device. Alternatively, one or more processing may be performed on the electrodermal activity signals before the electrodermal activity signals are transmitted to the mobile terminal device or the server device. For instance, the processing module of the smart ring device may be configured to obtain emotion-related characteristic data based on the electrodermal activity signals, and send the obtained characteristic data to the mobile terminal device or the server device for processing, so as to obtain an emotion monitoring result. Alternatively, the processing module of the smart ring device may be configured to obtain an emotion monitoring result indicating whether the user has an emotional response based on the electrodermal activity signals, and send the emotion monitoring result to the mobile terminal device or the server device for output to the user, or for subsequent analysis and statistics. In some other embodiments, the smart ring device may further include an output module such as at least one of an audio output module, a haptic output module, or a display. Correspondingly, after the processing module obtains the emotion monitoring result, the emotion monitoring result may be output to the user through the output module.

The emotion monitoring result may indicate whether the user has an emotional response, such as excitement, calmness, anger, or sadness, etc. In some embodiments, the detected emotional responses may be accumulated to determine the emotional tendency or emotional summary of the user over a longer period, and the emotional tendency or emotional summary may be output to the user. Alternatively, a guidance or suggestion for improving the emotion of the user is further output. The guidance or suggestion may include, for example, engaging in one or more physical activities and/or mental activities, which is not limited in the present disclosure.

In some embodiments, the body composition sensing module may be electrically connected to the first electrode 30 and the second electrode 40, and obtain the body composition signals of the user through the first electrode 30 and the second electrode 40. Based on the body composition signals, a body composition measurement result of the user, such as a body fat measurement result or the like, may be obtained. The body composition sensing module may be based on a two-electrode mode, a four-electrode mode, an eight-electrode mode, etc. In an example, the smart ring device 100 may further include one or more third electrodes for body composition measurement, e.g., one or more third electrodes may be provided on the outer surface of the outer ring 11. When the user wears the smart ring device 100 on a finger, the first electrode 30 and the second electrode 40 contact the skin of the finger wearing the smart ring device 100, and the one or more third electrodes may contact an adjacent finger of the user, or contact the other hand of the user, thereby realizing body composition measurements based on multiple electrodes. In another example, the smart ring device 100 may perform the body composition measurements in conjunction with another electronic device, such as a wrist-worn device like a wristwatch or a mobile terminal device. For instance, a wristwatch includes at least one third electrode, and when the user wears both the smart ring device 100 and the wristwatch, or a finger of the user simultaneously contacts the first electrode 30 and the second electrode 40 on the smart ring device, and meanwhile the user contacts at least one third electrode on the wristwatch, the body composition measurement of the user may be achieved. In this case, the smart ring device 100 may send the detected body composition signals or signals obtained by performing one or more processing on the detected body composition signals to another electronic device, such as the electronic device collaborating with the smart ring device 100 in body composition measurement, so that the another electronic device obtains the body composition measurement result of the user based on the body composition signals received from the smart ring device and the body composition signals detected itself. Alternatively, the smart ring device 100 and the another electronic device may both transmit the detected body composition signals or signals obtained by performing one or more processing on the detected body composition signals to a mobile terminal device or a server device, so that the mobile terminal device or the server device obtains the body composition measurement result of the user based on signals received from the devices.

In some embodiments, the temperature sensing module 23 may be electrically connected to at least one of the first electrode 30 or the second electrode 40. The first electrode 30 and/or the second electrode 40 may be configured to conduct skin temperature to the temperature sensing module 23. The temperature sensing module 23 may be configured to acquire the temperature signals and transmit the temperature signals or signals obtained by performing one or more processing on the temperature signals to the mobile terminal device or the server device, so that the mobile terminal device or the server device obtains a body temperature monitoring result of the user. The body temperature monitoring result may include a skin temperature monitoring result or a core temperature monitoring result. Thus, when the mobile terminal device or the server device detects an abnormal body temperature of the user, the mobile terminal device may alert the user, further enhancing health monitoring effect for the user, which is beneficial for timely and effective health monitoring and early detection of abnormal health conditions.

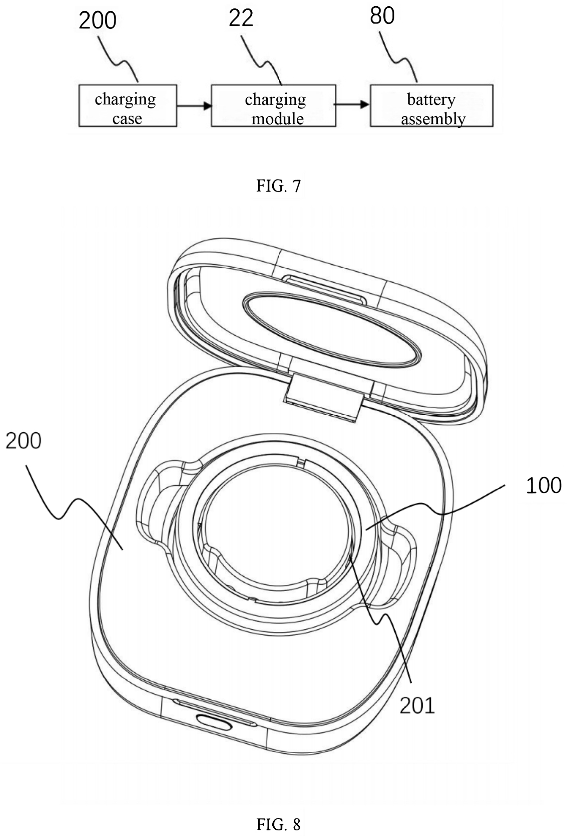

In some embodiments, the charging module 22 may be electrically connected to the first electrode 30 and the second electrode 40, receive electrical power through the first electrode 30 and the second electrode 40, and provide the electrical power to the battery assembly 80. As shown in the examples of FIGs. 7 and 8, a charging case 200 may include a base and a charging portion. When the smart ring device 100 is placed in the charging case 200, the first electrode 30 and the second electrode 40 are correspondingly connected to a plurality of charging terminals 201 of the charging portion, enabling the charging case 200 to charge the smart ring device 100. The charging module 22 may be based on wired charging or wireless charging, which is not limited in the present disclosure.

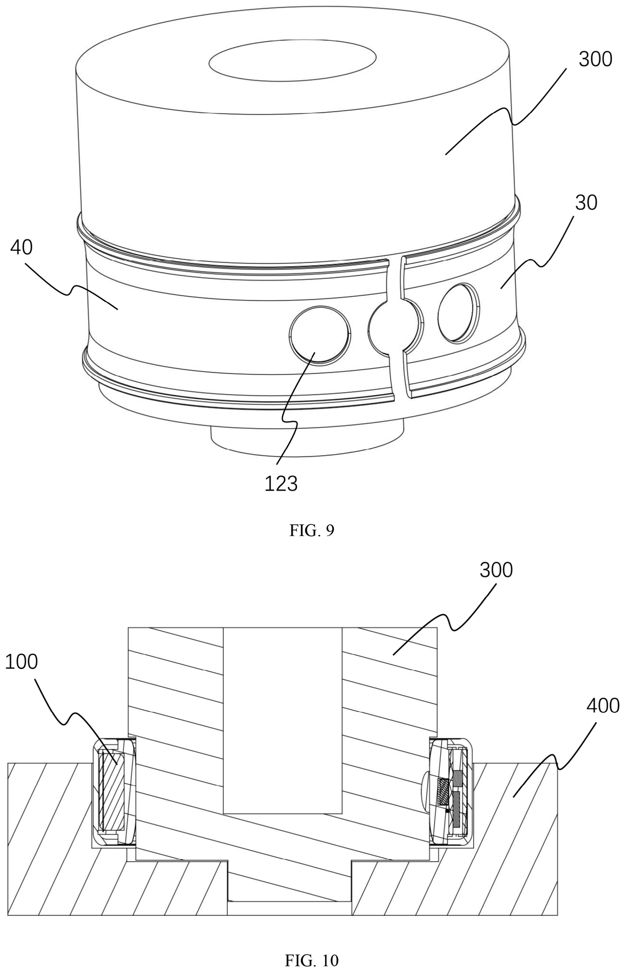

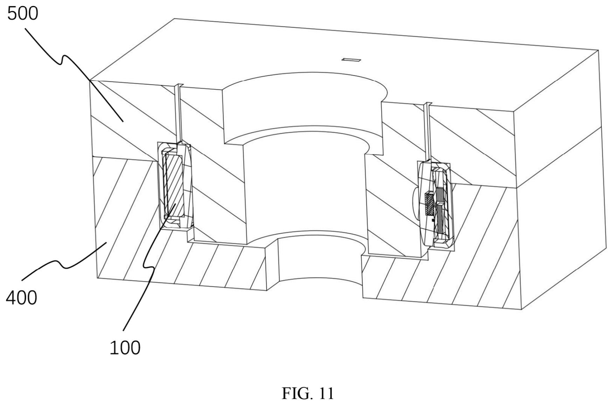

In some embodiments, fabrication of the smart ring device 100 may be achieved by potting. For example, the assembly and manufacturing of the smart ring device 100 may be achieved through a jig. As shown in the examples of FIGs. 9 to 11, the jig may include an upper positioning jig 300, a lower positioning jig 400, and a silicone jig 500. The manufacturing of the smart ring device 100 may be achieved through secondary potting.

First, the first electrode 30 and the second electrode 40 may be provided by vacuum suction with the upper positioning jig 300. Then, the upper positioning jig 300 with the first electrode 30 and the second electrode 40 is placed into the lower positioning jig 400, so as to achieve positioning of the bottom portion of the first electrode 30 and the second electrode 40. Then, adhesive dispensing is performed to bond the first electrode 30 and the second electrode 40 together. The bonding may be optionally achieved by potting of adhesive material. In some other embodiments, the first electrode 30 and the second electrode 40 may be assembled into an integral unit by an NMT injection molding process, and then bonded by potting of adhesive material after being positioned by the jigs.

Thereafter, the circuit board 70 and the curved battery assembly 80 may be assembled to the outer ring 11, and a conductive wire 73 is soldered to the first electrode 30 and the second electrode 40. Finally, instead of the upper positioning jig 300, the silicone jig 500 is used to pour epoxy resin into the smart ring device 100, thereby encapsulating the outer ring 11, the electronic components 20, the first electrode 30, the second electrode 40, the circuit board 70, and the curved battery assembly 80 together. The epoxy resin cures to form the isolation ring 60, and the inner surface of the isolation ring 60 protrudes between the first electrode 30 and the second electrode 40 to form the isolation component 50.

In the smart ring device 100 provided by the present disclosure, the first electrode 30 and the second electrode 40 electrically contact the charging terminals 201 in the charging case 200 to achieve the charging function. The first electrode 30 and the second electrode 40 in contact with the skin may obtain various signals from the user, including at least one of electrodermal activity signals, body composition signals or temperature signals. The first electrode 30 and the second electrode 40 may integrate the charging function and the function of obtaining physiological signals of the user, making the smart ring device 100 simple in structure, saving the manufacturing cost of the smart ring device 100, while making the assembly relatively simple and ensuring the aesthetics of the smart ring device 100.

The foregoing descriptions are only some embodiments of the present disclosure, and are not intended to limit the present disclosure in any form. Any modification, equivalent replacement, improvement, etc., made within the content of the present disclosure shall be included in the protection scope of the present disclosure.

## Claims

1. A smart ring device (100), comprising:
a housing (10) comprising an outer ring (11), an inner ring (12), and an accommodation space (13) between the outer ring (11) and the inner ring (12), wherein an outer surface of the housing (10) comprises at least a portion of an outer surface of the outer ring (11), and an inner surface of the housing (10) comprises at least a portion of an inner surface of the inner ring (12);
at least one electronic component (20) disposed within the accommodation space (13);
and
a first electrode (30) and a second electrode (40), spaced apart along a circumferential direction (C) of the inner surface of the housing (10) and electrically connected to the at least one electronic component (20).

2. The smart ring device (100) according to claim 1, wherein an isolation component (50) is provided between the first electrode (30) and the second electrode (40).

3. The smart ring device (100) according to claim 1 or 2, wherein:
each of the first electrode (30) and the second electrode (40) is in a shape of semi-annular or approximately semi-annular; and/or
at least one of the first electrode (30) or the second electrode (40) occupies more than two-thirds of the inner ring (12) in a width direction.

4. The smart ring device (100) according to claim 1, wherein:
a first aperture (121) and a second aperture (122) are symmetrically provided along a circumferential direction of the inner ring (12),
the first electrode (30) protrudes from the inner surface of the inner ring (12) through the first aperture (121), and
the second electrode (40) protrudes from the inner surface of the inner ring (12) through the second aperture (122).

5. The smart ring device (100) according to any one of claims 1 to 4, wherein an isolation ring (60) is provided between the outer ring (11) and the inner ring (12), the isolation ring (60) and the outer ring (11) enclosing to form the accommodation space (13).

6. The smart ring device (100) according to any one of claims 1 to 5, further comprising:
a circuit board (70) disposed within the accommodation space (13), wherein the circuit board (70) comprises at least two rigid circuit boards (71) and a flexible circuit board (72) connecting the at least two rigid circuit boards (71), and the at least one electronic component (20) is disposed on the at least two rigid circuit boards (71).

7. The smart ring device (100) according to claim 6, wherein:
an inner surface (111) of the outer ring (11) comprises at least one of a first mounting region (112) or a second mounting region (113),
at least a part of the at least two rigid circuit boards (71) is secured to the first mounting region (112) by a first locating structure, and
at least a part of the flexible circuit board (72) is secured to the second mounting region (113) by a second locating structure different from the first locating structure.

8. The smart ring device (100) according to claim 7, wherein:
the second mounting region (113) comprises a groove (1132) and a locating component (1131),
the flexible circuit board (72) comprises a mating component (721) adapted to the locating component (1131), and
one end of the locating component (1131) is fixedly disposed in the groove (1132), and the other end of the locating component (1131) is connected to the first electrode (30) or the second electrode (40) through the mating component (721).

9. The smart ring device (100) according to claim 7, wherein:
the second mounting region (113) comprises a locating post,
the flexible circuit board (72) comprises a locating aperture adapted to the locating post,
and
the locating post passes through the locating aperture and is fixedly connected to the first electrode (30) or the second electrode (40).

10. The smart ring device (100) according to any one of claims 1 to 5, further comprising:
a locating component (1131) at least partially disposed within the accommodation space (13) and a circuit board (70), wherein the circuit board (70) comprises a locating aperture adapted to the locating component (1131), one end of the locating component (1131) being connected to the outer ring (11), and the other end of the locating component (1131) being connected to the first electrode (30) or the second electrode (40) through the locating aperture on the circuit board (70).

11. The smart ring device (100) according to any one of claims 1 to 10, wherein the at least one electronic component (20) comprises an electrodermal activity sensing module (21) electrically connected to both the first electrode (30) and the second electrode (40).

12. The smart ring device (100) according to claim 11, wherein the at least one electronic component (20) further comprises at least one of a body composition sensing module, a charging module (22), or a temperature sensing module (23), and at least one of the body composition sensing module, the charging module (22), or the temperature sensing module (23) shares at least one of the first electrode (30) or the second electrode (40) with the electrodermal activity sensing module (21).

13. The smart ring device (100) according to any one of claims 1 to 3, further comprising an optical detection module (24) disposed within the accommodation space (13), the optical detection module (24) comprising a plurality of optical components and a plurality of convex lenses (241) located in correspondence with the plurality of optical components,
wherein a plurality of openings (123) are provided on the inner surface of the inner ring (12), and the plurality of convex lenses (241) protrude from the inner ring (12) through the plurality of openings (123), respectively.

14. The smart ring device (100) according to claim 2, further comprising an optical detection module (24) disposed within the accommodation space (13), the optical detection module (24) comprising at least one light emitter (243) as well as a first light detector (242) and a second light detector (244) disposed on two sides of the at least one light emitter, wherein the at least one light emitter (243) is configured to emit optical signals with at least two different wavelengths,
wherein a convex lens (241) corresponding to the at least one light emitter (243) is located in a region where the isolation component (50) is connected to the first electrode (30) and the second electrode (40).

15. The smart ring device (100) according to claim 1, further comprising an optical detection module (24) disposed within the accommodation space (13), wherein the first electrode (30) and the second electrode (40) are symmetrically disposed on opposite sides of the optical detection module (24).

16. The smart ring device (100) according to any one of claims 13 to 15, wherein the inner ring (12) is made from a light-transmitting material that allows light emitted and received by the optical detection module (24) to pass through.

17. The smart ring device (100) according to any one of claims 1 to 16, further comprising a circuit board (70) and a battery assembly (80), wherein the circuit board (70) and the battery assembly (80) are disposed in different regions within the accommodation space (13) along a circumferential direction of the housing (10).

18. The smart ring device (100) according to claim 17, wherein at least one first gap is formed between the circuit board (70) and the battery assembly (80), and at least one second gap is formed between the first electrode (30) and the second electrode (40), the at least one first gap and the at least one second gap being uniformly or approximately uniformly distributed along a circumferential direction of the housing (10).

19. The smart ring device according to any one of claims 1 to 18, wherein the first electrode (30) and the second electrode (40) are configured to detect electrodermal activity signals from finger skin of a user, and the electrodermal activity signals are used for determining whether the user has an emotional response.

20. The smart ring device according to claim 1, further comprising an optical detection module (24) disposed within the accommodation space (13), the optical detection module (24) comprising a plurality of optical components and a plurality of convex lenses (241) located in correspondence with the plurality of optical components,
wherein at least one protrusion is provided on the inner surface of the inner ring (12), and the plurality of convex lenses (241) comprise the at least one protrusion.
